(19) Europäisches Patentamt
European Patent Office
Office européen des brevets

(11) Publication number: **0 105 630**
**B1**

(12) **EUROPEAN PATENT SPECIFICATION**

(45) Date of publication of patent specification: **24.08.88**

(21) Application number: **83305164.2**

(22) Date of filing: **06.09.83**

(51) Int. Cl.⁴: **C 07 C 37/06**, C 07 C 62/32, C 12 P 7/42, C 12 N 1/20 // C12R1/40

# (54) Production of para-cresol.

(30) Priority: **07.09.82 US 415129**

(43) Date of publication of application:
**18.04.84 Bulletin 84/16**

(45) Publication of the grant of the patent:
**24.08.88 Bulletin 88/34**

(84) Designated Contracting States:
**BE DE FR GB IT NL**

(56) References cited:
**ZENTRALBL. BAKTERIOL., PARASITENKD., INFEKTIONSKR. HYG., ABT. 1: ORIG. REIHE B, vol. 162, 1976, pages 127-137; H.-J. KNACKMUSS et al.: "Zum Mechanismus der biologischen Persistenz von halogenierten und sulfonierten aromatischen Kohlenwasserstoffen"**

**JOURNAL OF BACTERIOLOGY, vol. 119, no. 3, September 1974, pages 923-929, American Society for Microbiology, US; J.F. DAVEY et al.: "Bacterial metabolism of para- and meta-xylene: oxidation of a methyl substituent"**

(73) Proprietor: **CELGENE CORPORATION**
**7 Powderhorn Drive**
**Warren New Jersey (US)**

(72) Inventor: **Hagedorn, Scott**
**Old Coach Road**
**Summit New Jersey (US)**

(74) Representative: **De Minvielle-Devaux, Ian Benedict Peter et al**
**CARPMAELS & RANSFORD**
**43, Bloomsbury Square**
**London WC1A 2RA (GB)**

(56) References cited:

**BIOCHEMISTRY, vol. 10, no. 13, 1971, pages 2530-2536, Easton, PA., US; A.M. REINER et al.: "Metabolism of benzoic acid by bacteria. Accumulation of (-)-3,5-cyclohexadiene-1,2-diol-1-carboxylic acid by a mutant strain of Alcaligenes eutrophus"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 105 630 B1

(56) References cited:
JOURNAL OF BACTERIOLOGY, vol. 119, no. 3, September 1974, pages 930-936, American Society for Microbiology, US; D.T. GIBSON et al.: "Bacterial metabolism of para- and meta-xylene: oxidation of the aromatic ring"

**Description**

The invention relates to the production of p-cresol. The invention provides the compound 4-methyl-cyclohexa-3,5-diene-1, 2-diol-carboxylic acid, a new micro-organism strain useful for producing the compound and a process for converting the compound into p-cresol.

p-Cresol is a speciality chemical with a market of 77 million pounds (34.93 million kilograms) per year. It is primarily employed in the synthesis of antioxidants such as butylated hydroxytoluenes, and in the preparation of thermosetting resins such as Resoles and Novolacs. Though cresols can be obtained directly from naphtha fractions of coal and oil it is difficult to separate the meta and para isomers due to their very similar boiling points. To obtain pure p-cresol, toluene is sulfonated with sulfuric acid followed by fusion with sodium hydroxide to yield a slurry that is rich in the para isomer. A final cryogenic crystallisation is employed to increase the purity of the p-cresol. Alternatively, toluene can be alkylated to a mixture of cymenes using propylene and a Lewis acid followed by dealkylation to cresol and a final cryogenic separation.

U.S. 2,437,648 describes a process for production of p-cresol which involves oxidation of toluene with hydrogen peroxide in the presence of a catalytically active metal oxide such as osmium tetraoxide.

U.S. 2,727,926 (Reissue 24,848) describes a process which involves converting toluic acid to p-cresol in the presence of a soluble copper catalyst such as copper benzoate.

U.S. 3,046,305 describes a process for converting monoalkylbenzene compounds to p-alkylphenols and p-alkylbenzoic acids which involves reacting a monoalkylbenzene with phosgene to provide p,p'-dialkylbenzophenone, and thereafter reacting the p,p'dialkylbenzophenone with hydrogen peroxide, acetic anhydride and sulfuric acid to yield p-alkylphenyl ester of p-alkylbenzoic acid, which intermediate is then hydrolysed to the corresponding p-alkylphenol and p-alkylbenzoic acid products.

Other patents which are of general interest include United States Patents 2,722,546; 2,903,480; 3,819,725; 3,929,911; 4,061,685; 4,189,602 and 4,277,012 and British Patent 964,980.

There is a continuing research effort to develop new and improved methods for the production of large volume speciality chemicals.

It is an object of this invention to provide a process for the production of p-cresol which is not energy intensive.

The invention surprisingly makes possible the efficient and energy-saving production of p-cresol, by means of a microbial metabolic pathway. This involves first the production of the compound 4-methyl-cyclohexa-3,5-diene-1,2-diol-carboxylic acid from p-xylene, by a microbial metabolic pathway, this compound being a p-cresol precursor. The latter may thereafter be converted to p-cresol.

The invention accordingly involves the surprising realisation that the compound 4-methylcyclohexa-3,5-diene-1,2-diol-carboxylic acid is readily obtainable by a microbial metabolic pathway from p-xylene (or other non-growth carbon source) and which can readily be converted to p-cresol.

Accordingly the invention involves the production of the p-cresol precursor 4-methylcyclohexa-3,5-diene-1,2-diol-carboxylic acid from a non-growth carbon source, especially p-xylene; a micro-organism which may be used for such production; and the conversion of the p-cresol precursor into p-cresol.

Other advantages of the present invention will become apparent from the following description and Examples.

The compound (p-cresol precursor) provided by the process of the invention is 4-methylcyclohexa-3,5-diene-1,2-diol-carboxylic acid. It may be produced from the non-growth carbon source, especially from p-xylene, by means of a micro-organism. An advantageous new micro-organism for use in this way is *Pseudomona putida* Biotype A strain ATCC No: 39119.

The compound may be used to provide p-cresol in a process for the production of p-cresol which comprises providing a reaction medium comprising an aqueous solution of 4-methylcyclohexa-3,5-diene-1,2-diol-1-carboxylic acid, and controlling the acidity of the aqueous solution so that its pH is sufficient to convert the said carboxylic acid compound to p-cresol. The controlling of the acidity may involve the addition of an acid.

The conversion of 4-methylcyclohexa-3,5-diene-1,2-diol-1-carboxylic acid to p-cresol is illustrated by the following reaction scheme:

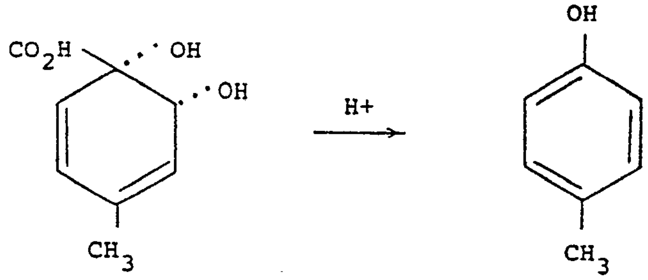

The aqueous medium optionally can contain a water-miscible organic solvent, for example ethanol, tetrahydrofuran or acetone.

The acidic pH of the reaction medium preferably is less than 6.5, and most preferably is less than 3. The reaction temperature will generally be in the range from 0°—100°C. Normally the reaction proceeds readily at ambient temperatures. The temperature has an effect on the degree of acidity required to ensure that the reaction proceeds readily, so that it is possible to some extent to compensate for a less low pH by raising the temperature.

However, it is one of the significant advantages of the invention that the reaction can readily be carried out at ambient temperatures simply by adjusting the pH.

The 4-methylcyclohexa-3,5-diene-1,2-diol-1-carboxylic acid in the aqueous reaction medium converts rapidly and essentially quantitatively at room temperature when the acidity of the aqueous reaction medium is adjusted to less than 3. A mineral acid such as hydrochloric acid is a convenient reagent for acidification of the aqueous reaction medium.

As demonstrated in Example II, at a neutral pH of about 7 the 4-methylcyclohexa-3,5-diene-1,2-diol-1-carboxylic acid compound is stable and does not spontaneously convert into p-cresol. If the same aqueous medium is heated to a temperature above 60°C, then the decomposition of carboxylic acid compound to p-cresol occurs.

The recovery of p-cresol from the aqueous medium can be accomplished by conventional means, e.g., by extraction with a water-immiscible solvent such as benzene or ethyl acetate.

An alternative method of recovering p-cresol when present in a low concentration is by contacting the aqueous medium with an adsorbent such as activated charcoal. Subsequently the adsorbed p-cresol can be stripped from the adsorbent substrate by washing with a desorbing agent, for example an alkanol (e.g. methanol) or an alkaline solution. The product adsorption and adsorbent regeneration procedures described in U.S. 4,071,398 can be applied for p-cresol recovery in the process of the present invention.

The 3-methylcyclohexa-3,5-diene-1,2-diol-1-carboxylic acid starting material described herein is related in chemical structure to a novel type of compounds which have been proposed in the literature as microbial cell culture metabolic intermediates having spectral characteristics consistent with a dihydrodiol configuration.

A dihydrodiol of an aromatic carboxylic acid was first reported in Biochemistry *10*, (13), 2530 (1971). A mutant strain of *Alcaligenes eutrophus* blocked in benzoic acid catabolism converted benzoic acid into a compound which has been assigned the structure 3,5-cyclohexadiene-1,2-diol-1-carboxylic acid:

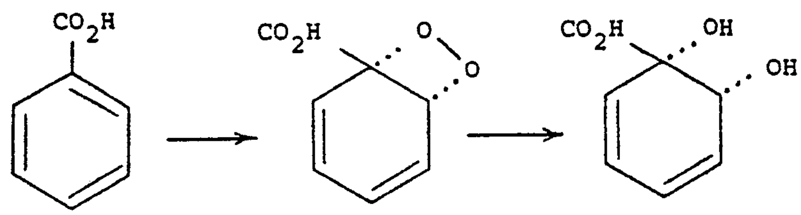

The same publication reported further that the proposed dihydrodiol compound illustrated above decomposed when an acidic aqueous solution of the compound was warmed to 45°C:

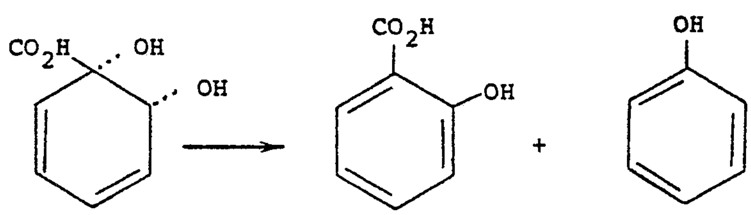

Further, a mutant strain of *Alcaligenes eutrophus*, when induced with benzoic acid, oxidised m-toluic acid to a product which exhibited spectra consistent with the structure 3-methylcyclohexa-3,5-diene-1,2-diol-1-carboxylic acid. In addition, an enzyme prepared from the wild-type organism catalysed the conversion of the proposed 3-methylcyclohexa-3,5-diene-1,2-diol-1-carboxylic acid to 3-methylcatechol.

A study of a bacterial metabolism of p-xylene and m-xylene is reported in Journal of Bacteriology, *119*(3), 923 (1974). *Pseudomonas* Pxy-40, when grown on succinate in the presence of p-xylene, accumulated p-toluic acid in the culture medium. Under the same conditions *Pseudomonas* Pxy-82 accumulated p-toluic acid and also 4-methylcatechol. By analogy to the Biochemistry, *10*(13) 2530 (1971) experimental results previously described hereinabove, a metabolic pathway was proposed for the initial reactions utilised by *Pseudomonas* Pxy to oxidise p-xylene. The pathway was visualised as proceeding via p-xylene to p-methylbenzyl alcohol to p-tolualdehyde to p-toluic acid to 4-methylcyclohexa-3,5-diene-1,2-diol-1-carboxylic acid to 4-methylcatechol. The latter 4-methylcatechol metabolite was isolated and

identified. There was no accumulation or identification of the presumed 4-methylcyclohexa-3,5-diene-1,2-diol-1-carboxylic acid metabolic intermediate.

Zbl. Bakt. Hyg., 1. Abt. Orig. B, *162*, 127—137 (1976) by Knackmuss et al describes the relative reaction rates of the oxygenation of substituted benzoates by *Pseudomonas putida* mt-2(m- or p-toluate utilizing) and *Pseudomonas strain* B13 (3-chlorobenzene utilizing); and the substituent effects in the dehydrogenation of 3,5-cyclohexadiene-1,2-diol-1-carboxylic acids.

Thus, with respect to the microbiological literature reviewed above, in the first case a 3,5-cyclohexadiene-1,2-diol-1-carboxylic acid metabolite converted to salicylic acid and phenol. In the second case, the presumed 4-methylcyclohexa-3,5-diene-1,2-diol-1-carboxylic acid compound was a transitory metabolite intermediate which converted *in vivo* to 4-methylcatechol. It was unexpected that (as provided by the present invention) 4-methylcyclohexa-3,5-diene-1,2-diol-1-carboxylic acid could be converted rapidly and quantitatively to p-cresol.

As described more fully in Example I below a microorganism was employed to convert p-xylene to a metabolic intermediate, i.e., 4-methylcyclohexa-3,5-diene-1,2-diol-1-carboxylic acid, which accumulated in the culture medium.

The micro-organism employed was a strain isolated from soil for its ability to grow on toluene. This strain also grows on p-xylene via p-toluate and 4-methylcatechol as metabolic intermediates. Species of *Pseudomonas* isolated from soil are frequently found to possess plasmids carrying the genes coding for the oxidation of toluene to central metabolites. Plasmids are separate pieces of circular DNA which are smaller than the chromosomal DNA. The particular plasmid of interest has been termed the TOL plasmid, and it codes for the degradation of toluene, p-xylene, m-xylene, benzoate, p-toluate and m-toluate.

The basal medium employed for purposes of the present invention to accumulate and provide the desired 4-methylcyclohexa-3,5-diene-1,2-diol-1-carboxylic acid starting material generally corresponded to that described in Biochem. Biophys. Res. Comm., *36*, 179 (1969). p-Xylene was introduced into the culture medium employing sterilised propylene vials.

As a general procedure, strains demonstrating mutant characteristics as determined by the inability to grow on p-xylene were grown on succinate, and then were given p-xylene. The strains of interest were those that appeared to accumulate the greatest amount of 4-methylcyclohexa-3,5-diene-1,2-diol-1-carboxylic acid without the accumulation of 4-methylcatechol.

By means of a chemostat, a strain of p-xylene oxidising *Pseudomonas* was isolated which exhibited an increased growth rate. The upgraded strain grew with a doubling time which was seven times shorter than that of the parent strain.

This new strain is fully identified as *Pseudomonas putida* Biotype A strain ATCC No: 39,119.

The following Examples are further illustrative of the present invention. The reactants and other specific ingredients and conditions are presented as being typical, and various modifications within the scope of the invention can be devised in the light of the foregoing disclosure.

## Example I

This Example illustrates the microbial culture accumulation of 4-methylcyclohexa-3,5-diene-1,2-diol-1-carboxylic acid from para-xylene metabolism.

The micro-organism is *Pseudomonas putida* Biotype A which is isolated from soil by enrichment for growth on toluene. The same micro-organism also has the ability to grow on p-xylene, m-xylene, p-toluate and benzoate (ATCC No: 39,119).

A neutral pH culture medium is provided which has the following composition:

| | |
|---|---|
| 50 mM | Na-K Phosphate Buffer |
| 2.0 g/litre | $NH_4Cl$ |
| 0.200 | $MgSO_4 \cdot 7H_2O$ |
| 0.001 | $FeSO_4 \cdot 7H_2O$ |
| 0.003 | $MnSO_4 \cdot H_2O$ |
| 0.003 | $ZnSO_4 \cdot 7H_2O$ |
| 0.001 | $CoSO_4 \cdot 7H_2O$ |
| 0.010 | EDTA |

A 250 ml Erlenmeyer flask containing 50 ml of the above minimal salts phosphate buffered pH 7.0 sterile liquid culture medium is inoculated with the above-described organism from a nutrient agar culture. P-xylene is added to a sterilised polypropylene liquid nitrogen storage vial which is then placed in the inoculated flask. When growth is observed as evidenced by an increase in cell turbidity, a sample is

withdrawn from the growing liquid culture, centrifuged and the supernatant is analysed for metabolic products.

The metabolic product 4-methylcyclohexa-3,5-diene-1,2-diol-1-carboxylic acid is characterised by the following properties:

*UV spectrum*

$\lambda max = 265$ nm

$\varepsilon = 6{,}000\ cm^{-1}M^{-1}$

*High Performance Liquid Chromatography (HPLC)*

Retention Time = 5.00—5.15 minutes

Conditions of HPLC:

Column: C18 Radial Compression HPLC Column (Waters Associates)

Solvent: 5% Isopropanol and 10 mM Phosphoric Acid

Flow Rate: 3 ml/min

The assignment of a vicinal-dihydrodiol configuration to the identified metabolite product is consistent with the pertinent literature. Bacterial catabolism initiated by double hydroxylation yielding cis-configurated dihydrodiols is described in Crit.Rev. Microbiol., *1*, 199 (1971) and Tetrahedron, *34*, 1707 (1978).

Example II

This Example illustrates the invention process for conversion of 4-methylcyclohexa-3,5-diene-1,2-diol-1-carboxylic acid to p-cresol, and effect of pH and temperature on the decomposition reaction.

An aqueous solution of 4-methylcyclohexa-3,5-diene-1,2-diol-1-carboxylic acid is divided into sample portions. The acidification of selected samples is accomplished with phosphoric acid. The heating of selected samples is at a temperature of about 80°C for 5 minutes. In the following tabulation, the minus sign represents absence of the compound in the final product medium, and the plus sign represents the presence of the compound in the final product medium.

| | Dihydrodiol* | p-Cresol |
|---|---|---|
| pH 2.0 | − | + |
| pH 7.0 | + | − |
| pH 10.0 | + | − |
| pH 7.0 + heat | − | + |
| pH 10.0 + heat | + | − |
| pH 10 + heat + readjustment to pH 2.0 | − | + |

* Dihydrodiol is determined by HPLC using a C18 column and 5% isopropanol + 10 mM phosphoric acid as a solvent (retention time, 5.00—5.15 minutes at a flow rate of 3 ml/min).

Activated charcoal is found to adsorb greater than 99 percent of the p-cresol in a 10 mM aqueous solution. About 35—40 percent of the adsorbed p-cresol is desorbed and recovered by contacting the charcoal with methanol.

As explained above, the conversion of the 4-methylcyclohexa-3,5-diene,1,2-diol-1-carboxylic acid to p-cresol takes place at a pH which depends on the temperature. While it may be possible to produce p-cresol in some cases by maintaining an unchanged pH and increasing the temperature, the preferred process involves lowering the pH, especially to less than 3.

The micro-organism of the invention, *Pseudomonas putida* Biotype A strain ATCC No: 39,119 has the following properties. It is a fluorescent *Pseudomonad*, Gram-negative, strictly aerobic, polarly flagellated and motile, and catalase positive.

It does not grow at temperatures above about 32°C. It grows on p-xylene, m-xylene, toluene, benzoate, p-toluic acid and m-toluic acid. It does not accumulate poly(beta-hydroxybutyrate). It metabolises glucose via 2-keto-3-deoxy-6-phosphogluconate. It exhibits toluate oxygenase activity.

## Claims

1. A process for the production of p-cresol which comprises providing a reaction medium comprising an aqueous solution of 4-methylcyclohexa-3,5-diene-1,2-diol-1-carboxylic acid, and controlling the acidity of the aqueous solution so that its pH is sufficient to convert the said carboxylic acid compound to p-cresol.

2. A process in accordance with claim 1 wherein the aqueous solution of 4-methylcyclohexa-3,5-diene-1,2-diol-1-carboxylic acid is derived by microbiological conversion of p-xylene; wherein the said conversion is effected by means of *Pseudomonas putida* Biotype A strain ATCC No: 39,119.

3. A process in accordance with any of claims 1—2 wherein the conversion of 4-methylcyclohexa-3,5-diene-1,2-diol-1-carboxylic acid to p-cresol is conducted at a pH of less than 3.

4. A process in accordance with any of claims 1—3 wherein the conversion of 4-methylcyclohexa-3,5-diene-1,2-diol-1-carboxylic acid to p-cresol is conducted at a temperature of 0°—100°C.

5. A process in accordance with any of claims 1—4 wherein the p-cresol product is removed from the aqueous medium by contact of the aqueous medium with a solid adsorbent.

6. A process in accordance with claim 5 wherein the p-cresol is subsequently desorbed from the solid adsorbent with alkanol.

7. *Pseudomonas putida* Biotype A strain ATCC No: 39,119.

## Patentansprüche

1. Verfahren zur Gewinnung von p-Cresol, umfassend das Bereitstellen eines eine wäßrige Lösung von 4-Methylcyclohexa-3,5-dien-1,2-diol-1-carbonsäure enthaltenden Mediums und die Regulierung der Acidität der wäßrigen Lösung in der Weise, daß deren pH ausreicht, um die genannte Carbonsäure-Verbindung in p-Cresol umzuwandeln.

2. Verfahren nach Anspruch 1, bei dem die wäßrige Lösung der 4-Methylcyclohexa-3,5-dien-1,2-diol-1-carbonsäure der mikrobiologischen Umwandlung von p-Xylol entstammt, wobei die Umwandlung mit Hilfe des Pseudomonas putida Biotyp A-Stammes ATCC Nr, 39 119 erfolgt.

3. Verfahren nach irgendeinem der Ansprüche 1 bis 2, bei dem die Umwandlung der 4-Methylcyclohexa-3,5-dien-1,2-diol-1-carbonsäure in p-Cresol bei einem pH von weniger als 3 durchgeführt wird.

4. Verfahren nach irgendeinem der Ansprüche 1 bis 3, bei dem die Umwandlung der 4-Methylcyclohexa-3,5-dien-1,2-diol-1-carbonsäure in p-Cresol bei einer Temperatur von 0°C bis 100°C durchgeführt wird.

5. Verfahren nach irgendeinem der Ansprüche 1 bis 4, bei dem das p-Cresol-Produkt aus dem wäßrigen Medium durch Kontakt des wäßrigen Mediums mit einem festen Adsorptionsmittel entfernt wird.

6. Verfahren nach Anspruch 5, bei dem das p-Cresol anschließend mit Alkanol von dem festen Adsorptionsmittel desorbiert wird.

7. Pseudomonas putida Biotyp A-Stamm ATCC Nr. 39 119.

## Revendications

1. Procédé pour la production du p-crésol qui consiste à produire un milieu réactionnel comprenant une solution aqueuse d'acide 4-méthylcyclohexa-3,5-diène-1,2-diol-1-carboxylique et à contrôler l'acidité de la solution aqueuse de manière que son pH soit suffisant pour convertir ledit composé d'acide carboxylique en p-crésol.

2. Procédé selon la revendication 1 où la solution aqueuse de l'acide 4-méthylcyclohexa-3,5-diène-1,2-diol-1-carboxylique est dérivée par conversion microbiologique du p-xylène; où ladite conversion est effectuée au moyen de *Pseudomonas putida* Biotype A souche ATCC N° 39 119.

3. Procédé selon l'une des revendications 1—2 où la conversion de l'acide 4-méthylcyclohexa-3,5-diène-1,2-diol-1-carboxylique en p-crésol est entreprise à un pH de moins de 3.

4. Procédé selon l'une des revendications 1—3 où la conversion de l'acide 4-méthylcyclohexa-3,5-diène-1,2-diol-1-carboxylique en p-crésol est entreprise à une température de 0°—100°C.

5. Procédé selon l'une des revendications 1—4 où le p-crésol produit est éliminé du milieu aqueux par contact du milieu aqueux avec un adsorbant solide.

6. Procédé selon la revendication 5 où le p-crésol est subséquemment désorbé de l'adsorbant solide avec de l'alcanol.

7. *Pseudomonas putida* Biotype A souche ATCC N° 39 119.